# EUROPÄISCHE PATENTANMELDUNG

(11) **EP 0 584 396 A1**
(43) Veröffentlichungstag der Anmeldung: **02.03.1994**
(21) Anmeldenummer: 92114796.3
(22) Anmeldetag: 28.08.1992
(51) Int. Cl.: A61M 39/00

(54) **Steckverbinder zum Herstellen und Unterbrechen einer Strömungsverbindung**

(71) Anmelder: Siemens Elema AB, S-171 95 Solna 1 (SE); SIEMENS AKTIENGESELLSCHAFT, D-80333 München (DE)
(72) Erfinder: von Witzleben, Dietrich, W-2800 Bremen 1 (DE)

(57) **Zusammenfassung**

Um bei einem Steckverbinder mit zwei Steckerteilen (1,2) zum Herstellen und Unterbrechen einer Strömungsverbindung beim Unterbrechen der Strömungsverbindung eine Abdichtung der voneinander getrennten Strömungszweige (3,8) zu ermöglichen, sind beide Steckerteile (1,2) jeweils mit einem Durchstichseptum (7,12) abgeschlossen, wobei in einem Septum (12) eine Hohlnadel (15) längsverschiebbar gehalten ist. Die Hohlnadel (15) und die Septa (7,12) sind in Bezug aufeinander derart ausgebildet, daß das die Hohlnadel (15) haltende Septum (12) dieser eine geringere Widerstandskraft gegen Durchstechen entgegensetzt, als das andere Septum (7). Dadurch wird erreicht, daß beim Zusammenfügen der Steckerteile (1,2) die Hohlnadel zuerst das sie haltende Septum (12) und danach das andere Septum (7) durchdringt und daß beim Lösen der Steckerteile (1,2) die Hohlnadel (15) zuerst aus dem sie haltenden Septum (12) und dann aus dem anderen Septum (7) herausgezogen wird.

## Beschreibung

Die Erfindung betrifft einen Steckverbinder zum Herstellen und Unterbrechen einer Strömungsverbindung insbesondere in medizinischen Apparaten, mit einem ersten Steckerteil, dass einen einseitig von einem Durchstichseptum abgeschlossenen Strömungsdurchlass aufweist, mit einem zweiten Steckerteil, dass einen in einem Verschlussteil endenden Strömungskanal enthält und eine in dem Verschlussteil angeordnete und dieses durchdringende Hohlnadel aufweist, und mit Mitteln zum lösbaren Verbinden beider Steckerteile, wobei das Durchstichseptum in dem ersten Steckerteil und die Hohlnadel in dem Zweiten Steckerteil derart angeordnet sind, dass bei zusammengefügten Steckerteilen die Hohlnadel das Durchstichseptum durchdringt und eine Strömungsverbindung zwischen dem Strömungsdurchlass und dem Strömungskanal herstellt.

Bei einem derartigen, aus der US-A-4 950 260 bekannten Steckverbinder weist das erste von zwei Steckerteilen einen mit einem Durchstichseptum abgeschlossenen Flüssigkeitseinlass auf, während das zweite Steckerteil eine mit einem Flüssigkeitsauslass verbundene Hohlnadel aufweist, die in einem Verschlussteil und von einer Hülse umgeben fest angeordnet ist. Beim Zusammenfügen der beiden Steckerteile durchdringt die Kohlnadel das Durchstichseptum und stellt so einen Flüssigkeitsdurchlass zwischen dem Flüssigkeitseinlass und dem Flüssigkeitsauslass her. Die Hülse des zweiten Steckerteils dient zum Zentrieren beider Steckerteile, wenn diese zusammengefügt werden. Ausserdem schützt die Hülse die Hohlnadel, wenn beide Steckerteile voneinander getrennt sind. Da bei dem bekannten Steckverbinder nur das erste Steckerteil mit einem Dichtungsmittel in Form des Durchstichseptums versehen ist, ist der bekannte Steckverbinder nur dann anwendbar, wenn der Flüssigkeitstransport in eine einzigen Richtung, nämlich von dem ersten zu dem zweiten Steckerteil erfolgen soll.

Der Erfindung liegt daher die Aufgabe zugrunde, einen Steckverbinder anzugeben, der bei entkoppelten Steckerteilen unabhängig von der Strömungsrichtung eine sichere Abdichtung der beiden voneinander getrennten Strömungszweige ermöglicht.

Gemäss der Erfindung wird die Aufgabe dadurch gelöst, dass bei dem Steckverbinder der eingangs angegebenen Art das Verschlussteil aus einem zweiten Durchstichseptum besteht, in dem die Hohlnadel in Richtung ihrer Längsausdehnung verschiebbar gehalten ist, dass die Hohlnadel und die Durchstichsepten in Bezug aufeinander derart ausgebildet sind, dass das zweite Durchstichseptum der Hohlnadel eine geringere Widerstandskraft entgegensetzt als das erste Durchstichseptum, dass das zweite Steckerteil Anschläge zur Begrenzung der Verschiebung der Hohlnadel in dem zweiten Steckerteil zwischen einer ersten und einer zweiten Stellung aufweist, wobei die Hohlnadel in der ersten Stellung das zweite Durchstichseptum nur zu einem Teil durchdringt und in der zweiten Stellung völlig durchdringt, und dass die Länge der Hohlnadel derart bemessen ist, dass sie in ihrer zweiten Stellung bei zusammen gefügten Steckerteilen auch das erste Durchstichseptum vollständig durchdringt.

Beim Zusammenfügen der beiden Steckerteile wird die Hohlnadel zunächst von dem Durchstichseptum des ersten Steckerteils durch das zweite Durchstichseptum in dem zweiten Steckerteil hindurchgedrückt bevor die Hohlnadel auch das erste Durchstichseptum in dem ersten Steckerteil durchdringt. Umgekehrt wird beim Lösen der beiden Steckerteile die Hohlnadel zunächst aus dem zweiten Durchstichseptum des zweiten Steckerteil soweit herausgezogen, dass sich das zweite Durchstichseptum selbsttätig verschliesst; erst dann wird das erste Durchstichseptum des ersten Steckerteils von der Hohlnadel abgezogen. Auf diese Weise ist sichergestellt, dass beide Steckerteile stets nach aussen hin abgedichtet sind, so dass weder beim Herstellen noch beim Lösen der Steckverbindung beispielsweise Aussenluft in die Strömungswege des Steckverbinders eindringen kann.

Um zu erreichen, dass beim Zusammenfügen der Steckerteile die Hohlnadel zunächst das zweite Durchstichseptum in dem zweiten Steckerteil durchdringt bzw. beim Lösen der Steckverbindung zuerst aus diesem herausgezogen wird, ist in vorteilhafter Weise vorgesehen, dass die Durchstichsepten aufgrund unterschiedliche Materialeigenschaften unterschiedliche Reibungswerte gegenüber der sie durchdringenden Hohlnadel aufweisen. Dies kann durch unterschiedliche Materialsorten oder unterschiedliche Materialdichten erreicht werden. Alternativ oder ergänzend hierzu ist im Rahmen der Erfindung vorgesehen, dass die Hohlnadel aufgrund unterschiedlicher Form und/oder Oberflächenbeschaffenheit an ihren beiden Enden unterschiedliche Reibungswerte gegenüber den Durchstichsepten aufweist. So kann beispielsweise das in dem zweiten Durchstichseptum liegende Ende der Hohlnadel spitzer als das andere Ende ausgebildet sein, um so das zweite Durchstichseptum leichter durchdringen zu können.

Entsprechend einer vorteilhaften Weiterbildung des erfindungsgemässen Steckverbinders weist die Hohlnadel an ihrer das erste Durchstichseptum durchdringenden Spitze eine Öffnung mit einem senkrecht zur Längsausdehnung der Hohlnadel verlaufenden Öffnungsrand auf. Dadurch wird erreicht, dass sich beim Zusammenfügen der beiden Steckerteile das Durchstichseptum des ersten Steckerteils dichtend an den Öffnungsrand anlegt und verhindert, dass Aussenluft in die Hohlnadel einströmen kann, wenn diese das zweite Durchstichseptum in dem zweiten Steckerteil durchstösst und so eine Verbindung zu dessen Innenraum herstellt.

Bei einer besonders vorteilhaften Weiterbildung des erfindungsgemässen Steckverbinders ist das zweite Steckerteil in Form einer Hülse ausgebildet die an einem Ende offen und an dem anderen Ende durch das Verschlussteil mit der in das Innere der Hülse hineinragenden Hohlnadel abgeschlossen ist, wobei die Hülse beim Zusammenfügen beider Steckerteile das erste Steckerteil nach aussen hin abdichtend umfasst. Dabei ist die Länge der Hülse so bemessen, dass beim Zusammenfügen der beiden Steckerteile diese dichtend ineinander greifen, bevor die Hohlnadel das zweite Durchstichseptum in dem zweiten Steckerteil durchdringt. Auf diese Weise ist sichergestellt, dass auch dann keine Aussenluft in das Innere des zweiten Steckerteils eindringt, wenn beispielsweise aufgrund eines Bedienungsfehlers das Durchstichseptum des ersten Steckerteils nicht richtig dichtend an der Öffnung der Hohlnadel anliegt.

In diesen Zusammenhang ist von besonderem Vorteil, wenn die Hohlnadel eine seitliche Öffnung aufweist, die in Richtung der Längsausdehnung der Hohlnadel derart angeordnet ist, dass sie in der ersten Stellung der Hohlnadel im Inneren der Hülse liegt und der zweiten Stellung von dem zweiten Durchstichseptum dichtend umschlossen wird.

Der erfindungsgemässe Steckverbinder erweist sich insbesondere im Zusammenhang mit dem Ein- oder Nachfüllen von flüssigen Medikamenten in implantierbare Medikamentendosiergeräten von Vorteil. Bekannte implantierbare Medikamentendosiergeräte (DE-A-36 05 664) z. B. für Insulin, Zytostatika oder Schmerzmittel weisen einen Medikamentenspeicher auf, der in bestimmten, vom spezifischen Medikamentenbedarf des Patienten abhängigen zeitlichen Intervallen nachgefüllt werden muss. Üblicherweise erfolgt diese Nachfüllung transkutan mit einer Injektionsspritze deren Kanüle durch die Haut und ein darunter liegendes, den Medikamentenspeicher nach aussen hin abdichtendes Nachfüllseptum hindurchgestochen wird. Um vor einem Wiederauffüllen des Medikamentenspeichers zunächst Medikamentenreste aus diesem heraussaugen zu können, ist entsprechend einer Weiterbildung der Erfindung vorgesehen, dass das erste Steckerteil mit dem zu entleerenden Flüssigkeitsspeicher verbunden ist und dass an dem zweiten Steckerteil ein Absaugbehälter angeschlossen ist, in dessen Inneren ein geringerer Druck als in dem Flüssigkeitsspeicher vorliegt. Sobald die Steckverbindung zwischen den beiden Steckerteilen hergestellt ist, werden die Flüssigkeitsreste aus dem Flüssigkeitsspeicher heraus in den Absaugbehälter hineingesaugt. Wenn die beiden Steckerteile wieder voneinander gelöst werden, ist sowohl der Flüssigkeitsspeicher als auch der Absaugbehälter jeweils nach aussen hin abgedichtet.

Zum Wiederbefüllen des Flüssigkeitsspeichers ist im Rahmen der Erfindung das erste Steckerteil mit dem zufüllenden Flüssigkeitsspeicher verbunden und an dem zweiten Steckerteil ein flüssigkeitsgefüllter Füllbehälter angeschlossen, in dessen Inneren ein höherer Druck als in dem Flüssigkeitsspeicher vorliegt.

Im Zusammenhang mit dem transkutanen Ein- oder Nachfüllen von flüssigen Medikamenten in implantierbare Medikamentendosiergeräte ist in vorteilhafter Weise an dem ersten Steckerteil eine Kanüle zum Durchstechen eines den Flüssigkeitsspeicher nach aussen hin abdichtenden Nachfüllseptums angeschlossen. Dadurch wird eine sichere Nachfüllung ermöglicht, wobei insbesondere die Gefahr eines Abbrechens, Abknickens oder Herausrutschens der Kanüle aus der Einstichstelle und damit verbunden die Gefahr von Fehlinjektionen vermieden wird.

Zur Erläuterung der Erfindung wird im folgendem auf die Figuren der Zeichnung Bezug genommen; im einzelnen zeigen
- Fig. 1 bis 7: ein Ausführungsbeispiel des erfindungsgemässen Steckverbinders in mehreren Phasen beim Zusammenfügen und Entkoppeln der beiden Steckerteile und
- Fig. 8: ein weiteres Ausführungsbeispiel des erfindungsgemässen Steckverbinders zum Entleeren und Wiederbefüllen eines implantierten Medikamentendosiergerätes.

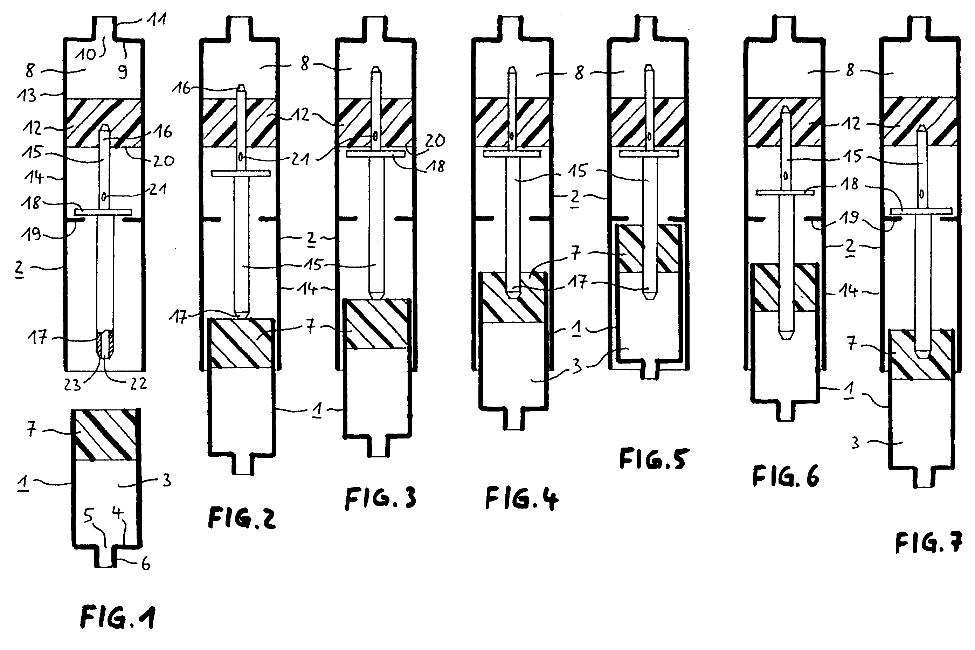

Die Figuren 1 bis 7 zeigen jeweils in einer längsgeschnittenen Seitenansicht einen Steckverbinder bestehend aus einem ersten Steckerteil 1 und einem zweiten Steckerteil 2 in aufeinanderfolgenden Phasenabschnitten beim Zusammenfügen und anschliessenden Entkoppeln beider Steckerteile 1 und 2. Das erste Steckerteil 1 enthält einen Strömungsdurchlass 3 in Form einer topfförmigen Kammer, die im Bereich des Topfbodens 4 eine Öffnung 5 für den Durchlass eines Strömungsmediums aufweist. Im Bereich der Öffnung 5 ist das Steckerteil 1 mit einem Anschlussteil 6 zum Anschluss einer hier nicht gezeigten Leitung für das Strömungsmedium versehen. Die gegenüberliegende offene Seite der Kammer 3 ist durch ein Durchstichseptum 7 verschlossen.

Das zweite Steckerteil 2 enthält einen Strömungskanal 8 ebenfalls in Form einer topfförmigen Kammer, die im Bereich des Topfbodens 9 eine Öffnung 10 für den Durchlass des Strömungsmediums enthält und ebenfalls mit einem Anschlussteil 11 zum Anschluss einer Leitung für das Strömungsmedium versehen ist. Auf der gegenüberliegenden Seite ist die Kammer 8 durch ein Verschlussteil 12 in Form eines zweiten Durchstichseptums abgedichtet, dass ebenso wie das erste Durchstichseptum 7 vorzugsweise aus Bromobutylgummi besteht. Die Wandung 13 der Kammer 8 ist über das zweite Durchstichseptum 12 hinaus zu einer Hülse 14 verlängert, deren innere Weite im wesentlichen der äusseren Weite des ersten Steckerteils 1 entspricht, so dass die Hülse 14 beim Zusammenfügen der beiden Steckerteile 1 und 2 das erste Steckerteil 1 nach aussen hin abdichtend umfasst.

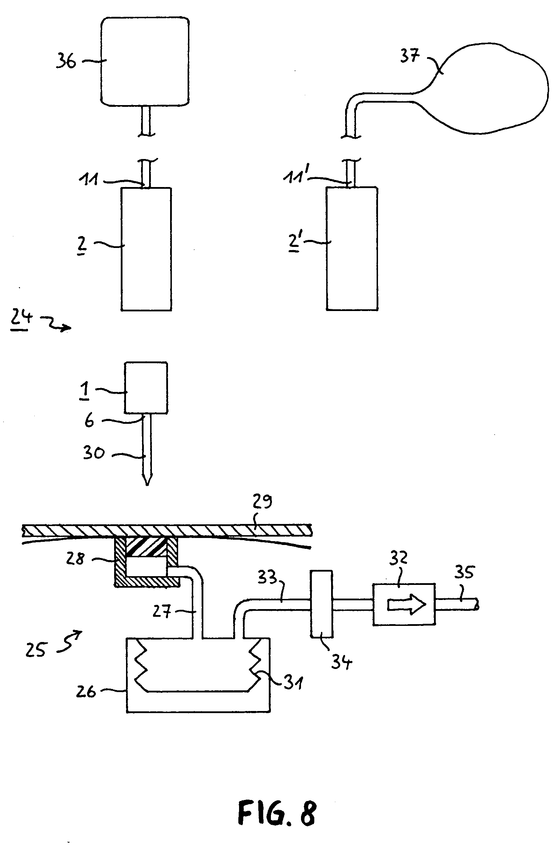

Das zweite Durchstichseptum 12 dient als Halterung für eine Hohlnadel 15, die sich im Inneren der Hülse 14 in Längsrichtung zu dieser erstreckt und an einem Ende 16 in den zweiten Durchstichseptum 12 steckt, während das andere Ende 17 der Hohlnadel 15 im Bereich der Öffnung der Hülse 14 liegt. Die Hohlnadel 15 ist in Richtung ihrer Längsausdehnung verschiebbar angeordnet, wobei sie das Durchstichseptum 12 mehr oder weniger durchdringt. Die Hohlnadel 15 weist einen hier ringförmigen Ansatz 18 auf, durch den ihre Bewegung zwischen zwei Anschlägen 19 und 20 des zweiten Steckerteils 2 begrenzt ist. Dabei sind die Anschläge 19 und 20 derart angeordnet, dass in einer ersten, in den Figuren 1 und 7 gezeigten Stellung, in der die Hohlnadel 15 mit ihrem Ansatz 18 an dem mit 19 bezeichneten Anschlag anliegt, das zweite Durchstichseptum 15 von dem Ende 16 der Hohlnadel 15 nur zu einem Teil durchdrungen wird, so dass die Hohlnadel 15 an dieser Stelle abgedichtet ist. Wenn die Hohlnadel 15 in einer zweiten, in den Figuren 3, 4 und 5 gezeigten Stellung mit ihrem Ansatz an dem von dem zweiten Durchstichseptum 12 gebildeten zweiten Anschlag 20 anliegt, wird das zweite Durchstichseptum 12 vollständig von der Hohlnadel 15 durchdrungen, so dass über die Hohlnadel 15 eine Verbindung mit dem Inneren der Kammer 8 besteht. Die Hohlnadel 15 weist ferner eine seitliche Öffnung 21 auf, die in Richtung der Längsausdehnung der Hohlnadel 15 derart angeordnet ist, dass sie in der ersten Stellung (Fig. 1 und 7) im Inneren der Hülse liegt und in der zweiten Stellung (Figuren 3, 4 und 5) von dem zweiten Durchstichseptum 12 dichtend umschlossen wird.

Die Länge der Hülse 14 ist so bemessen, dass sie bei der ersten Stellung (Fig. 1 und 7) der Hohlnadel 15 über deren Spitze 17 hinausragt, so dass diese weitgehend gegen eine mögliche Beschädigung durch unsachgemässe Handhabung geschützt ist. Ausserdem wird dadurch beim Zusammenfügen der Steckerteile 1 und 2 das erste Steckerteil 1 gegenüber der Hohlnadel 15 in einer festen Lage fixiert, bevor die Hohlnadel 15 mit ihrer Spitze 17 auf das Durchstichseptum 7 trifft.

Die beiden Durchstichsepten 7 und 8 und die Hohlnadel 15 sind in bezug aufeinander derart ausgebildet, dass das zweite Durchstichseptum 12 der Hohlnadel 15 beim Durchdringen eine geringere Widerstandskraft entgegen-setzt, als das erste Durchstichseptum 7. Dies kann auf unterschiedliche Weise realisiert werden, indem z.B. die beiden Durchstichsepten 7 und 12 aufgrund verschiedener Materialsorten oder Materialdichten gegenüber der sie durchdringenden Hohlnadel 15 eine unterschiedlich hohe Reibung aufweisen. Alternativ oder ergänzend hierzu kann die Hohlnadel 15 aufgrund unterschiedlicher Form und/oder Oberflächenbeschaffenheit an ihren beiden Enden 16 und 17 unterschiedliche Reibungswerte gegenüber den Durchstich-septen 7 und 12 aufweisen. So kann beispielsweise die Hohlnadel 15 an ihrem in dem zweiten Durchstichseptum 12 liegenden Ende 16 einen geringeren Aussendurchmesser und eine glattere Oberfläche aufweisen, als an dem anderen Ende 17. Wie Fig. 1 zeigt, ist die Öffnung 22 der Hohlnadel 15 an ihrer das erste Durchstichseptum 7 durchdringenden Spitze 17 mit einem senkrecht zur Längsausdehnung der Hohlnadel 15 verlaufenden Öffnungsrand 23 ausgebildet, so dass beim Zusammenfügen der beiden Steckerteile 1 und 2 das erste Durchstichseptum 7 zunächst dichtend an der Öffnung 22 anliegt ( Figuren 2 und 3).

Fig. 1 zeigt den Steckverbinder bei voneinander entkoppelten Steckerteilen 1 und 2.

Wie Fig. 2 zeigt, wird beim Zusammenfügen der beiden Steckerteile 1 und 2 zunächst das erste Steckerteil 1 durch die Hülse 14 gegenüber der Hohlnadel 15 zentriert. Gleichzeitig wird das Innere der Hülse 14 nach aussen hin von dem Steckerteil 1 abgedichtet. Beim weiteren Zusammenfügen der beiden Steckerteile 1 und 2 legt sich das erste Durchstichseptum 7 dichtend an die Spitze 17 der Hohlnadel 15 an und schiebt diese mit ihren anderen Ende 16 durch das zweite Durchstichseptum 12 hindurch. Wenn die Hohlnadel 15 soeben das zweite Durchstichseptum 12 durchdrungen hat, befindet sich die seitliche Öffnung 21 der Hohlnadel 15 immer noch im Inneren der Hülse 14, so dass dieses über die Hohlnadel 15 mit den Strömungskanal 8 des zweiten Steckerteils 2 in Verbindung steht und leergesaugt werden kann.

Entspechend der Darstellung in Fig. 3 wird beim weiteren Zusammenschieben der Steckerteile 1 und 2 die Hohlnadel 15 von dem ersten Durchstichseptum 7 soweit d urch das zweite Durchstichseptum 12 hindurchgedrückt, bis der Ansatz 18 an dem von dem zweiten Durchstichseptum 12 gebildeten Anschlag 20 anliegt. In dieser Stellung der Hohlnadel 15 wird deren seitliche Öffnung 21 von dem zweiten Durchstichseptum 12 dichtend umschlossen, so dass keine Verbindung mehr zwischen dem Strömungskanal 8 und dem Inneren der Hülse 14 besteht.

Wie die Figuren 4 und 5 zeigen, wird beim weiteren Zusammenschieben der beiden Steckerteile 1 und 2 die Hohlnadel 15 mit ihrem Ende 17 in das Durchstichseptum 7 des ersten Steckerteils 1 hineingedrückt bis die Hohlnadel 15 das erste Durchstichseptum 7 völlig durchdringt und eine Strömungsverbindung zwischen dem Strömungsdurchlass 3 des ersten Steckerteils 1 und dem Strömungskanal 8 des zweiten Steckerteils 2 herstellt.

Beim Entkoppeln der beiden Steckderteile 1 und 2 wird entsprechend Fig. 6 die Hohlnadel 15 zunächst soweit aus dem Durchstichseptum 12 des zweiten Steckerteils 2 herausgezogen, bis der Ansatz 18 der Hohlnadel 15 an den Anschlag 19 zu liegen kommt. In dieser Stellung der Hohlnadel 15 ist der Strömungskanal 8 des zweiten Steckerteils 2 gegenüber dem Inneren der Hohlnadel 15 und der Hülse 14 durch das selbstheilende Durchstichseptum 12 abgedichtet.

Wie Fig. 7 schliesslich zeigt, wird beim weiteren Ent-fernen der beiden Steckerteile 1 und 2 voneinander die Hohlnadel 15 auch aus dem Durchstichseptum 7 des ersten Steckerteils herausgezogen, wobei das Durchstichseptum 7 den Strömungsdurchlass 3 nach aussenhin abdichtet.

Der erfindungsgemässe Steckverbinder ist also sehr einfach zu handhaben, wobei sichergestellt ist, dass beide Steckerteile 1 und 2 stets nach aussenhin abgedichtet sind, so dass weder beim Herstellen noch beim Lösen der Steckverbindung beispielsweise Aussenluft in die Strömungswege des Steckverbinders eindringen kann.

Fig. 8 zeigt ein weiteres Ausführungsbeispiel des erfindungsgemässen Steckverbinders 24 im Zusammenhang mit dem Nachfüllen von flüssigen Medikamenten in ein implantierbares Medikamentendosiertgerät 25. Bei dem hier nur teilweise und vereinfacht dargestellen implantierten Medikamentendosiergerät 25 ist ein Medikamentenspeicher 26 über einen Einfüllschlauch 27 mit einem Nachfüllseptum 28 verbunden, welches unter der Haut 29 eines Lebewesens so angeordnet ist, dass ein flüssiges Medikament, wie z.B. Insulin mittels einer Kanüle 30 durch das Nachfüllseptum 28 hindurch in ein Membranbalgreservoir 31 des Medikamentenspeichers 26 nach dessen Entleerung einfüllbar ist. Eine Medikamentendosierpumpe 32 saugt das gespeicherte Medikament über einen Verbindungsschlauch 33 gegebenfalls durch ein Filter 34 hindurch in dosierten Portionen ab und führt diese einem Katheter 35 zu, der die Medikamentenportionen an eine geeignete Stelle im Körper des Lebewesens transportiert und appliziert. Die Kanüle 30 ist als Bestandteil des ersten Steckerteils 1 an dessen Anschlussteil 6 angeschlossen. Für das Entleeren und Wiederauffüllen des Medikamentenspeichers 26 sind jeweils zwei zweite Steckerteile 2 und 2' vorgesehen, von denen das mit 2 bezeichnete Steckerteil an seinem Anschlussteil 11 mit einem Absaugbehälter 36 verbunden ist, in dessen Inneren ein geringerer Druck, als in dem Medikamentenspeicher 26 vorliegt; das mit 2' bezeichnete zweite Steckerteil ist an seinem Anschlussteil 11' mit einem das nachzufüllende Medikament beinhaltenden Füllbehälter 37 verbunden.

Das Entleeren und Wiederbefüllen des Medikamentenspeichers 26 geschieht in der Weise, dass zunächst mit der Kanüle 30 des ersten Steckerteils 1 die Haut 29 und das darunterliegende Nachfüllseptum 28 durchstochen wird und so ein Zugang zu dem Medikamentenspeicher 26 geschaffen wird. Zum Absaugen von Restflüssigkeiten in dem Medikamentenspeicher 26 wird zunächst das mit 2 bezeichnete zweite Steckerteil auf das erste Steckerteil 1 aufgesetzt, so dass durch den Unterdruck in dem Absaugbehälter 36 die gesamte Anordnung evakuiert und das Restvolumen des Medikaments aus dem Medikamentenspeicher 26 entfernt wird, wobei sich das Membranbalgreservoir 31 zusammenzieht. Anschliessend wird das mit 2 bezeichnete zweite Steckerteil von dem ersten Steckerteil 1 abgezogen, wobei das Durchstichseptum 7 (vgl. Fig. 1 bis 6) gewährleistet, das der Unterdruck in der Anordnung aufrecht erhalten bleibt.

Zum Wiederbefüllen des Medikamentenspeichers 26 wird das mit 2' bezeichnete Steckerteil auf das erste Steckerteil 1 aufgesetzt, wobei das in dem Füllbehälter 37 enthaltene frische Medikament durch den in der Anordnung herrschenden Unterdruck in den Medikamentenspeicher 26 eingesaugt wird, bis nach völligen Einsaugen ein Druckausgleich eintritt. Wie bereits an Hand der Figuren 1 bis 7 beschrieben, ist sowohl beim Entleeren als auch beim Nachfüllen des Medikamentenspeichers 26 stets sichergestellt, dass keine Aussenluft in das Medikamentendosiergerät 25 eindringen kann.

### Bezugszeichenliste

- 1,2,2': Steckerteile
- 3: Strömungsdurchlaß
- 4: Topfboden
- 5: Öffnung
- 6: Anschlußteil
- 7: Durchstichseptum
- 8: Strömungskanal
- 9: Topfboden
- 10: Öffnung
- 11,11': Anschlußteile
- 12: Verschlußteil (Durchstichseptum)
- 13: Wandung von 8
- 14: Hülse
- 15: Hohlnadel
- 16,17: Enden von 15
- 18: Ansatz
- 19,20: Anschläge
- 21: seitliche Öffnung in 15
- 22: Öffnung an 17
- 23: Öffnungsrand
- 24: Steckverbinder
- 25: implantierbares Medikamentendosiergerät
- 26: Medikamentenspeicher
- 27: Einfüllschlauch
- 28: Nachfüllseptum
- 29: Haut
- 30: Kanüle
- 31: Membranbalgreservoir
- 32: Medikamentendosierpumpe
- 33: Verbindungsschlauch
- 34: Filter
- 35: Katheter
- 36: Absaugbehälter
- 37: Füllbehälter

## Patentansprüche

1. Steckverbinder zum Herstellen und Unterbrechen einer Strömungsverbindung insbesondere in medizinischen Apparaten, mit einem ersten Steckerteil (1), das einen einseitig von einem Durchstichseptum (7) abgeschlossenen Strömungsdurchlass (3) aufweist, mit einem zweiten Steckerteil (2), das einen in einem Verschlussteil (12) endenden Strömungskanal (8) enthält und eine in dem Verschlussteil (12) angeordnete und dieses durchdringende Hohlnadel (15) aufweist, und mit Mitteln zum lösbaren Verbinden beider Steckerteile (1, 2), wobei das Durchstichseptum (7) in dem ersten Steckerteil (1) und die Hohlnadel (15) in dem zweiten Steckerteil (2) derart angeordnet sind, dass bei zusammengefügten Steckerteilen (1, 2) die Hohlnadel (15) das Durchstichseptum (7) durchdringt und eine Strömungsverbindung zwischen dem Strömungsdurchlass (3) und dem Strömungskanal (8) herstellt, **dadurch gekennzeichnet**, dass das Verschlussteil (12) aus einem zweiten Durchstichseptum besteht, in dem die Hohlnadel (15) in Richtung ihrer Längsausdehnung verschiebbar gehalten ist, dass die Hohlnadel (15) und die Durchstichsepten (7, 12) in Bezug aufeinander derart ausgebildet sind, dass das zweite Durchstichseptum (12) der Hohlnadel (15) eine geringere Widerstandskraft entgegensetzt als das erste Durchstichseptum (7), dass das zweite Steckerteil (2) Anschläge (19, 21) zur Begrenzung der Verschiebung der Hohlnadel (15) in dem zweiten Steckerteil (2) zwischen einer ersten (Fig. 1, 7) und einer zweiten (Fig. 3, 4, 5) Stellung aufweist, wobei die Hohlnadel (15) in der ersten Stellung das zweite Durchstichseptum (12) nur zu einem Teil durchdringt und in der zweiten Stellung völlig durchdringt, und dass die Länge der Hohlnadel (15) derart bemessen ist, dass sie in ihrer zweiten Stellung bei zusammengefügten Steckerteilen (1, 2) auch das erste Durchstichseptum (7) vollständig durchdringt.

2. Steckerverbinder nach Anspruch 1, **dadurch gekennzeichnet**, dass die Durchstichsepten (7, 12) aufgrund unterschiedlicher Materialeigenschaften unterschiedliche Reibungswerte gegenüber der sie durchdringenden Hohlnadel (15) aufweisen.

3. Steckverbinder nach Anspruch 1 oder 2, **dadurch gekennzeichnet**, dass die Hohlnadel (15) aufgrund unterschiedlicher Form und/oder Oberflächen-beschaffenheit an ihren beiden Enden (16, 17) unter-schiedliche Reibungswerte gegenüber den Durchstichsepten (7, 12) aufweist.

4. Steckverbinder nach einem der vorangehenden Ansprüche, **dadurch gekennzeichnet**, dass die Hohlnadel (15) an ihrer das erste Durchstichseptum (7) durchdringenden Spitze (17) eine Öffnung (22) mit einem senkrecht zur Längsausdehnung der Hohlnadel (15) verlaufenden Öffnungsrand (23) aufweist.

5. Steckverbinder nach einem der vorangehenden Ansprüche, **dadurch gekennzeichnet**, dass das zweite Steckerteil (2) in Form einer Hülse (14) ausgebildet ist, die an einem Ende offen und and dem anderen Ende durch das Verschlussteil (12) mit der in das Innere der Hülse (14) hineinragenden Hohlnadel (15) abge-schlossen ist, und dass die Hülse (14) beim Zusammenfügen beider Steckerteile (1, 2)das erste Steckerteil (1) nach aussen hin abdichtend umfasst.

6. Steckverbinder nach Anspruch 5**, dadurch gekennzeichnet**, dass die Hohlnadel (15) eine seitliche Öffnung (21) aufweist, die in Richtung der Längsausdehnung der Hohlnadel (15) derart angeordnet ist, dass sie in der ersten Stellung der Hohlnadel (15) im Inneren der Hülse (14) liegt und in der zweiten Stellung von dem zweiten Durchstichseptum (12) dichtend umschlossen wird.

7. Steckverbinder nach einem der vorangehenden Ansprüche, **dadurch gekennzeichnet**, dass das erste Steckerteil (1) mit einem zu entleerenden Flüssigkeitsspeicher (26) verbunden ist und dass an dem zweiten Steckerteil (2) ein Absaugbehälter (36) angeschlossen ist, in dessen Inneren ein geringerer Druck als in dem Flüssigkeitsspeicher (26) vorliegt.

8. Steckverbinder nach einem der Ansprüche 1 bis 6, **dadurch gekennzeichnet**, dass das erste Steckerteil (1) mit einem zu füllenden Flüssigkeits-speicher (26) verbunden ist und dass an dem zweiten Steckerteil (2) ein flüssigkeitsgefüllter Füllbehälter (37) angeschlossen ist, in dessen Inneren ein höherer Druck als in dem Flüssigkeitsspeicher (26) vorliegt.

9. Steckverbinder nach Anspruch 7 oder 8, **dadurch gekennzeichnet**, dass an dem ersten Steckerteil (1) eine Kanüle (30) zum Durchstechen eines den Flüssigkeitsspeicher (26) nach aussen hin abdichtenden Nachfüllseptums (28) angeschlossen ist.
